(19) Europäisches Patentamt
European Patent Office
Office européen des brevets

(11) **EP 4 670 761 A1**

(12) **EUROPEAN PATENT APPLICATION**

(43) Date of publication:
**31.12.2025   Bulletin 2026/01**

(21) Application number: **24185252.4**

(22) Date of filing: **28.06.2024**

(51) International Patent Classification (IPC):
***A61M 5/315*** (2006.01)

(52) Cooperative Patent Classification (CPC):
**A61M 5/31513**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Becton, Dickinson and Company
Franklin Lakes, NJ 07417-1880 (US)**

(72) Inventors:
• **FLIPPE, Marc
38640 CLAIX (FR)**
• **VAXELAIRE, Jérémie
74100 Ambilly (FR)**
• **RODRIGUEZ SAN JUAN, Nestor
Hamburg, New Jersey, 07419 (US)**

(74) Representative: **Germain Maureau
12, rue Boileau
69006 Lyon (FR)**

(54) **TWO RIB SYRINGE PLUNGER STOPPER WITH AXIAL GUIDING FEATURE**

(57)     Provided herein is a stopper adapted for attachment with a plunger rod for use within a syringe barrel. The stopper includes a main body portion defining a proximal end and a distal end and having a main body axial length extending between the proximal and distal ends, with the proximal end configured to mate with a distal end of the plunger rod. The stopper also includes first and second ribs extending radially from an outer surface of the main body portion and around an outer circumference of the main body portion, with the second rib axially spaced from the first rib. The main body portion of the stopper includes an inter-rib region extending axially between the first rib and the second rib, characterized in that an inter-rib axial length of the inter-rib region is 30 percent or greater of the main body axial length.

**EP 4 670 761 A1**

## Description

## BACKGROUND OF THE INVENTION

Field of the Invention

The present disclosure relates generally to a plunger stopper for use with a syringe and, more particularly, to a plunger stopper with features thereon that provide improved axial guiding of the stopper within a syringe barrel.

Description of Related Art

Medical injection devices, such as syringes, are used in a variety of environments for administering liquids (e.g., medications or drugs) to a patient. Many syringes are provided as prefilled syringes, which provide the convenience of rapidly delivering the liquid therein to a patient without the need to first aspirate the medication from another container and meter its volume. For dispensing fluids, the prefilled syringe will typically include a syringe barrel with a plunger assembly inserted through an open proximal end of the barrel and an opening at the opposite distal end adapted to receive a needle therein by which a fluid is injected into the patient. The plunger assembly typically includes an elongated plunger rod extending out of the barrel, and a plunger head or stopper disposed at the distal end of the plunger rod. The stopper or plunger stopper is typically made of elastomeric material and is adapted to ensure the container closure integrity of a syringe when the stopper is inserted into the syringe.

Existing stopper designs include a stopper body having a tail portion disposed at its proximal end adapted for attachment to the distal end of the plunger rod, and a head portion disposed at its distal end adapted to interfit with the barrel of the syringe. The head portion of the stopper may include a plurality of annular, outwardly protruding ribs formed on an external cylindrical wall thereof, with the ribs forming a seal with the syringe barrel to ensure the container closure integrity of the syringe. A series of two or three ribs, for example, may be spaced apart longitudinally along the stopper main body, with each pair of adjacent ribs separated by an inter-rib region provided therebetween. The inter-rib region is generally formed to present a cylindrical outer surface on the stopper between the ribs that is inset radially inward from the ribs.

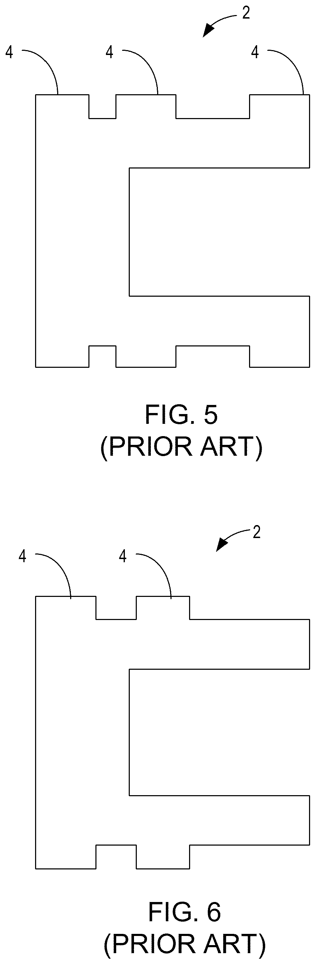

Existing stopper designs as described above - with the stopper including a series of two or three ribs - can present issues during use of the syringe and/or during insertion of the stopper into the syringe barrel, e.g., during a vent tube stoppering process. As shown in FIG. 5, for stoppers 2 including a series of three spaced apart ribs 4, the arrangement of three ribs can lead to increased friction between the stopper and the syringe barrel that makes advancement of the stopper within the syringe barrel (via pushing of plunger rod) more difficult. As shown in FIG. 6, for stoppers 2 including two spaced apart ribs 4, the two ribs are typically arranged close together (i.e., a small inter-rib distance or pitch, such as between 1 to 5 mm), with the arrangement of the two closely spaced ribs potentially leading to problems in axially guiding/aligning the stopper within the syringe barrel and guaranteeing proper sealing between the stopper and syringe barrel, so as to ensure leak resistance & container closure integrity (CCI). As relates to a vent tube stoppering process where an installation rod applies a pushing force to a center portion of the stopper for positioning of the stopper within the syringe barrel, the arrangement of the two closely spaced ribs increases the risk of the stopper tilting as it is being pushed out of the vent tube and into the syringe barrel - with the result being that the stopper may not form a proper seal with the container wall when pushed into position. As relates to use of the syringe where the plunger rod applies a pushing force to the stopper to advance the stopper within the syringe barrel, the arrangement of the two closely spaced ribs similarly increases the risk of the stopper tilting and at least one of the ribs not maintaining a sufficient contact pressure against the syringe barrel to form a proper seal therewith.

Accordingly, a need exists in the art for a plunger stopper for use with a syringe, where the stopper is constructed to reduce the amount of friction present between the stopper and the syringe barrel during use and/or a stopper insertion process, while still enabling proper axially guiding and aligning of the stopper within the syringe barrel to guarantee proper sealing between the stopper and syringe barrel.

## SUMMARY OF THE INVENTION

Provided herein is a stopper adapted for attachment with a plunger rod for use within a syringe barrel. The stopper includes a main body portion comprising a proximal end and a distal end and having a main body axial length extending between the proximal and distal ends, with the proximal end configured to mate with a distal end of the plunger rod to secure the stopper to the plunger rod. The stopper also includes a first rib extending radially from an outer surface of the main body portion and around an outer circumference of the main body portion, and a second rib axially spaced from the first rib, with the second rib extending radially from the outer surface of the main body portion and around the outer circumference of the main body portion. The main body portion of the stopper includes an inter-rib region extending axially between the first rib and the second rib, characterized in that an inter-rib axial length of the inter-rib region is 30 percent or greater of the main body axial length.

In certain configurations, the inter-rib axial length is preferably 70 percent or greater of the main body axial length.

In certain configurations, the first rib is a distal rib and the second rib is a proximal rib, with the inter-rib axial length comprising a pitch between a proximal edge of the distal rib and a distal edge of the proximal rib.

In certain configurations, the first rib and the second rib, and the pitch therebetween, forms a stabilizing feature configured to maintain an axial guiding and aligning of the stopper within the syringe barrel.

In certain configurations, each of the first rib and the second rib comprises a radially outward-directed contact surface, and wherein a width of the contact surface is between 0.2 mm and 10 mm and preferably between 0.2 mm and 1.5 mm.

In certain configurations, the first and second ribs are integrally formed with the main body portion, with each of the main body portion and the first and second ribs formed of a base material, the base material comprising rubber.

In certain configurations, the stopper further includes a laminate or coating applied to at least a radially outward-directed contact surface of the first rib and the second rib, the laminate or coating having a lower coefficient of friction than the base material.

In certain configurations, the laminate or coating comprises one or more of ultra-high-molecular-weight polyethylene (UHMWPE), liquid silicone rubber (LSR), polydicyclopentadiene (PDCPD), polymethyl urea (PMU), polyvinylidene difluoride (PVDF), polyvinyl, a polysulfone film, or a fluoropolymer.

Also provided herein is a syringe including a syringe barrel having a barrel proximal end and a barrel distal end and defining a chamber configured for containing a fluid therein, and a plunger assembly axially movable within the chamber of the syringe barrel between a retracted position and an advanced position, with the plunger assembly including a plunger having a plunger proximal end and a plunger distal end and a stopper secured to the plunger distal end, the stopper being free or substantially free of per- and poly- fluoroalkyl substances (PFAS). The stopper includes a main body portion defining a proximal end and a distal end, with the proximal end configured to mate with a distal end of the plunger rod to secure the stopper to the plunger rod. The stopper also includes a first rib extending from an outer surface of the main body portion and around an outer circumference of the main body portion, and a second rib spaced from the first rib, with the second rib extending from the outer surface of the main body portion and around the outer circumference of the main body portion. The stopper also includes an inter-rib region positioned between the first rib and the second rib, characterized in that the inter-rib region comprises a curved inter-rib region where the outer surface of the main body portion is curved radially inward.

In certain configurations, the inter-rib axial length is preferably 70 percent or greater of the main body axial length, so as to prevent tilting of the stopper when placed into or advanced through the syringe barrel.

In certain configurations, the syringe comprises a 10 mL syringe, with the main body axial length of the stopper being approximately 9 mm and the inter-rib axial length of the inter-rib region being between 6 and 8 mm.

In certain configurations, the syringe comprises a 5 mL syringe, with the main body axial length of the stopper being approximately 8.5 mm and the inter-rib axial length of the inter-rib region being between 5.5 and 7.5 mm.

In certain configurations, the syringe comprises a 1-3 mL syringe, with the main body axial length of the stopper being approximately 7.7 mm and the inter-rib axial length of the inter-rib region being between 5 and 7 mm.

In certain configurations, the stopper comprises a base material and a laminate or coating applied to at least a portion of an outer surface of the base material, wherein the base material comprises rubber, and wherein the laminate or coating comprises one or more of ultra-high-molecular-weight polyethylene (UHMWPE), liquid silicone rubber (LSR), polydicyclopentadiene (PDCPD), polymethyl urea (PMU), polyvinylidene difluoride (PVDF), polyvinyl, a polysulfone film, or a fluoropolymer.

In certain configurations, an interference between the first and second ribs and the syringe barrel is in the range of 1.5 to 20%, as determined by: Interference $= ((OD_{ribs}/ID_{barrel}) - 1) \times 100$, wherein $OD_{ribs}$ is an outer diameter of the first and second ribs and $ID_{barrel}$ is an inner diameter of the syringe barrel.

## BRIEF DESCRIPTION OF THE DRAWINGS

FIG. 1 is a perspective view of a syringe, with which embodiments of the disclosure may be implemented;

FIG. 2 is an exploded view of the syringe of FIG. 1;

FIG. 3 is a side view of a stopper included in the syringe of FIG. 1, according to a non-limiting embodiment described herein;

FIG. 4 is a side cross-sectional view of the stopper of FIG. 3 taken along line 4-4;

FIG. 5 is a side cross sectional view of a prior art stopper adapted for attachment with a plunger rod for use within a syringe barrel, as known in the art; and

FIG. 6 is a side cross sectional view of another prior art stopper adapted for attachment with a plunger rod for use within a syringe barrel, as known in the art.

## DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the present disclosure, the distal end of a component or of a device means the end furthest away from the hand of the user and the proximal end means the end closest to the hand of the user, when the component or device is in the use position. Thus, with a syringe for example, the distal end is the end thereof that includes a needle (or luer connection), while the proximal end is where the user engages the plunger (i.e., the plunger thumb press). Similarly, in this application, the terms "in the distal direction" and "distally" mean in the direction toward the distal tip of the syringe, and the terms "in the proximal direction" and "proximally" mean in the direction opposite the direction of the distal tip of the syringe (i.e., in the direction of the plunger thumb press).

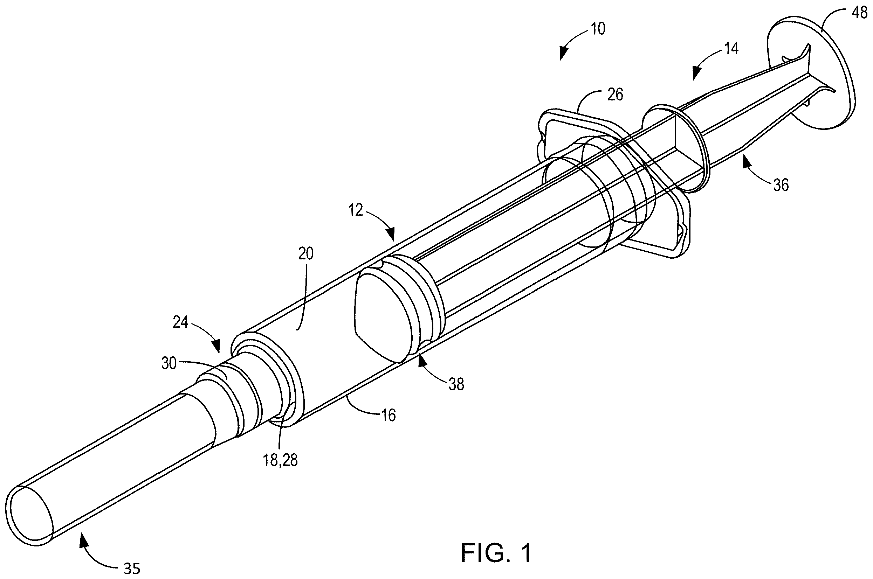

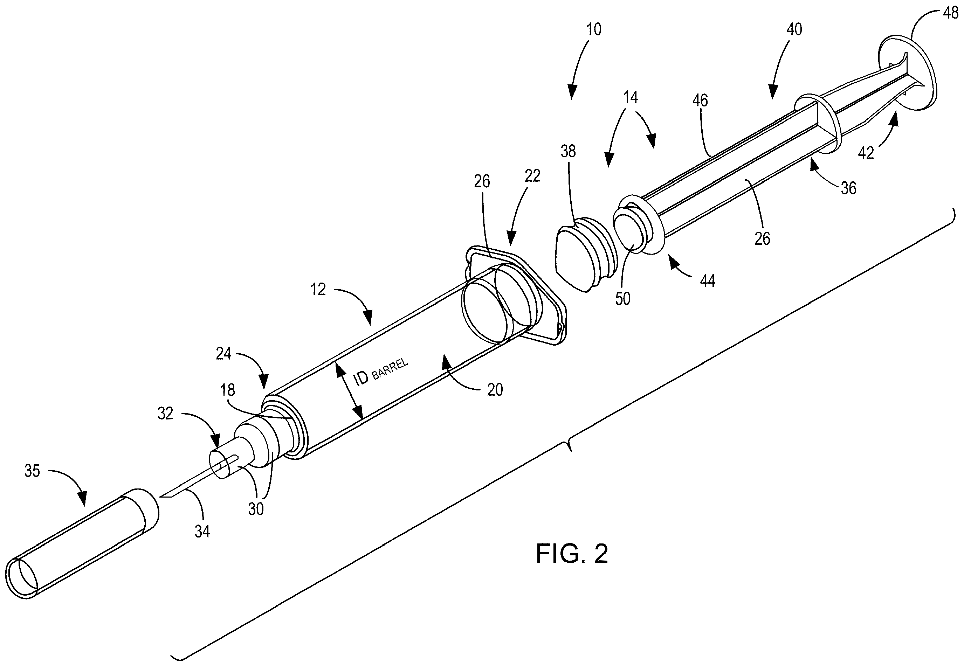

Referring to FIGS. 1 and 2, shown is a non-limiting embodiment of a syringe 10 with which aspects or embodiments of the disclosure may be implemented. According to some aspects of the disclosure, the syringe 10 may be provided as a prefilled syringe, which provides the convenience of rapidly delivering the liquid therein to a patient without the need to first aspirate the medication from another container and meter its volume.

As shown in FIGS. 1 and 2, the syringe 10 generally includes a syringe barrel 12 and a plunger assembly 14. The plunger assembly 14 is movable within the syringe barrel 12 along a longitudinal axis to an advanced position to facilitate administering of an injectable fluid (e.g., medication) to a patient, for example. The syringe barrel 12 is formed of a generally cylindrical outer wall 16 and an end member 18 that collectively define a chamber 20 for retaining fluid therein. The syringe barrel 12 includes an open proximal end 22 configured to receive the plunger assembly 14 therein and a distal end 24 at which end member 18 is positioned. The proximal end 22 of the syringe barrel 12 may include a flange 26 to facilitate handling and positioning of the syringe 10 and to maintain the relative position of the syringe barrel 12 with respect to the plunger assembly 14 during medication administration. At the distal end 24, the end member 18 may include a shoulder 28 which narrows with respect to the cylindrical outer wall 16, as well as a hub portion 30 extending out distally from shoulder 28. The hub portion 30 is formed as a partially hollow member that defines a channel 32 therethrough in fluid communication with the chamber 20. A needle 34 is attached to the hub portion 30 within channel 32, such as by being glued or otherwise secured to the hub portion 30. According to some aspects of the disclosure, syringe 10 may further include a cover 35 that couples to the hub portion 30 of syringe barrel 12 to protect the needle 34.

The plunger assembly 14 of syringe 10 is formed of an elongate plunger rod 36 (more generally "plunger 36," as used hereafter) and a plunger head or stopper 38. The plunger 36 may include a main body 40 extending between a plunger proximal end 42 and a plunger distal end 44. In some embodiments, the main body 40 may include a plurality of elongate vanes or walls 46 extending axially along a length thereof between the plunger proximal end 42 and the plunger distal end 44. A thumb press 48 is positioned at the plunger proximal end 42 that may be engaged by a thumb (or other finger) of the user to apply a distally directed force to the plunger assembly 14 to move the plunger 36 with respect to the syringe barrel 12. In some embodiments, an extension member 50 (e.g., threaded cylindrical member) is positioned at the plunger distal end 44 that is configured to mate with the stopper 38. In other embodiments, the plunger distal end 44 may include a female receptacle formed therein that is configured to receive and connect to a protrusion (e.g., pin) extending out proximally from the stopper 38, with the protrusion and receptacle engaging via threading, for example.

The stopper 38 of plunger assembly 14 is positioned at the plunger distal end 44 so as to be movable along with the plunger 36 within the chamber 20 of syringe barrel 12. That is, as a distally-directed force is applied to the plunger rod 36 (such as to thumb press 48), both the plunger rod 36 and the stopper 38 are caused to move distally through syringe barrel 12, with the stopper 38 configured to maintain a seal with an inner surface of the syringe barrel 12 as it is moved therethrough.

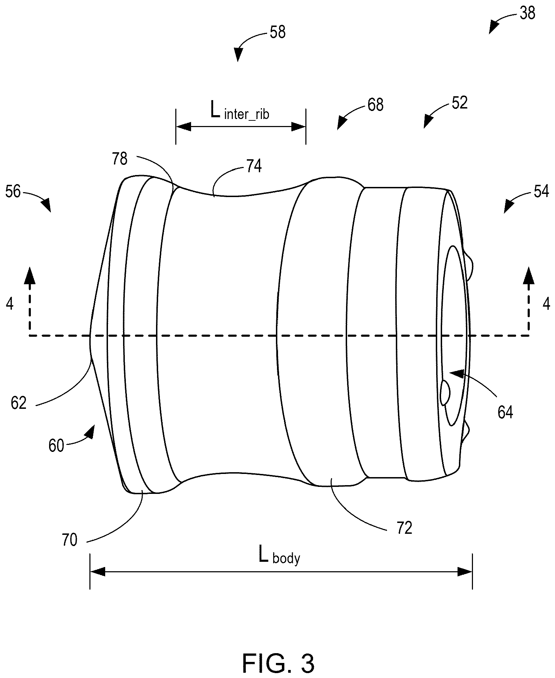

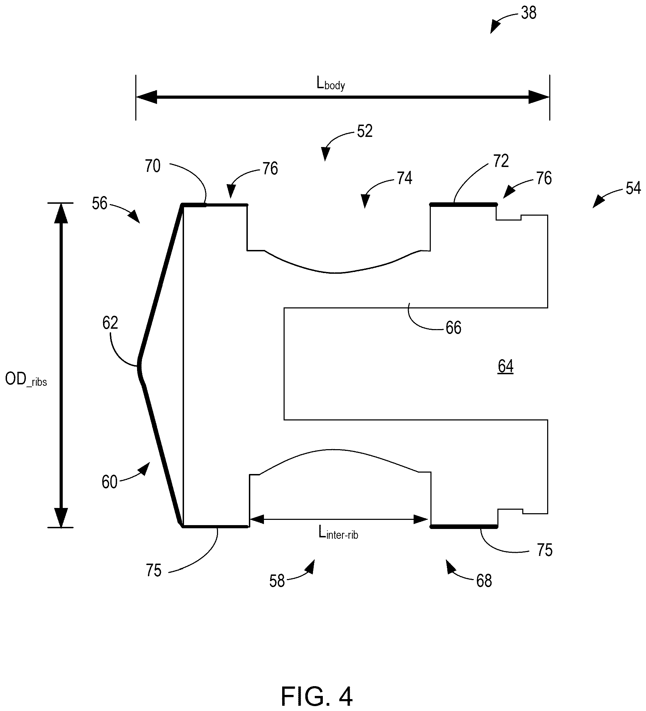

Referring now to FIGS. 3 and 4, and with continued reference to FIGS. 1 and 2, the structure of stopper 38 is shown in greater detail, according to an aspect or embodiment of the disclosure. The stopper 38 is defined by a main body 52 that includes a proximal end 54 and a distal end 56, with the main body 52 having an axial length, $L_{body}$, extending between the proximal and distal ends 54, 56. The proximal end 54 is configured as an open proximal end that engages with the distal end of plunger 36, while the distal end 56 is configured as a closed distal end configured to engage with the barrel 12 of syringe 10. The closed distal end 56 of the main body 52 includes a generally cylindrical portion 58 and a roof portion 60 that extends distally from cylindrical portion 58. In one embodiment, the roof portion 60 is configured as a conical portion that extends distally from cylindrical portion 58 to a tip 62 to provide the closed distal end 56. However, it is recognized that roof portion 60 may have other suitable shapes, such as being formed as a flat surface or domed surface, as other non-limiting examples.

As shown in FIG. 4, the main body 52 of the stopper 38 is partially hollow and is sized and configured to receive the extension member 50 of plunger 36 therein. The main body 52 of the stopper 38 defines an inner cavity 64 that is generally defined between a majority of the open proximal end 54 and the closed distal end 56 of the main body 52. In some embodiments, and as previously indicated, the inner cavity 64 includes a threaded inner surface 66 that is configured to receive and mate with a threaded extension member 50 of plunger 36. In other embodiments, the inner cavity 64 may define an inner contoured surface having a notch therein that is configured to receive a flanged extension member of plunger 36, with the flanged extension member engaging the notch in order to retain the extension member 50 within the inner cavity 64.

Referring still to FIGS. 3 and 4, the outer surface 68 of the cylindrical portion 58 of the main body 52 includes a plurality of ribs 70, 72 formed thereon that extend circumferentially around the entire outer surface 68 of the cylindrical portion 58. The ribs 70, 72 may be formed integrally with the main body 52, such as via a molding process. The plurality of ribs 70, 72 includes a first or distal rib 70 positioned toward the distal end 56 of the main body 52 and a second or proximal rib 72 positioned toward the proximal end 54 of the main body 52 - with the first rib 70 spaced apart longitudinally/axially from the second rib 72 on the cylindrical portion 58 to form an inter-rib region 74 therebetween. The inter-rib region 74 of cylindrical portion 58 may have a generally flat outer surface 68 or a curved outer surface 68.

The stopper 38 may be made from a material that is different from the material of the plunger 36 and that is capable of forming a tight seal with the syringe barrel 12 as it is advanced therethrough. In some embodiments, the stopper 38 is formed of an elastomeric material or rubber material such as butyl, styrene butadiene, isoprene, as non-limiting examples.

According to aspects of the disclosure, the stopper 38 may further include an outer laminate or coating 75 on at least portions thereof, such as on a sealant or gliding surface thereof - i.e., on the contact surface 76 of first rib 70 and second rib 72 - and/or on roof portion 60. The laminate/coating 75 may be configured to reduce friction between the stopper 38 and barrel 12 when the stopper 38 is moved within the syringe 10. According to aspects of the disclosure, the laminate/coating 75 may be formed of one or more of ultra-high-molecular-weight polyethylene (UHMWPE), liquid silicone rubber (LSR), polydicyclopentadiene (PDCPD), polymethyl urea (PMU), polyvinylidene difluoride (PVDF), polyvinyl, a polysulfone film, or a fluoropolymer, as non-limiting examples. In some embodiments, it may be desirable for the laminate/coating 75 to be formed of a material that is free or substantially free of per- and polyfluoroalkyl substances (PFAS) - generally, a "PFAS-free" material - that might leach into the medicament or other solution stored in pre-filled syringe 10. As defined herein, the laminate/coating 75 being "free or substantially free of PFAS" or "PFAS-free" is understood to refer to materials that are entirely free of PFAS and/or materials that may contain trace amounts of PFAS therein (e.g., 200 parts-per-million (PPM) or less of PFAS therein, and preferably 50 PPM or less of PFAS therein).

According to embodiments of the disclosure, the structure of each of first rib 70 and second rib 72 may be controlled to achieve a desired interaction between the stopper 38 and the barrel 12. According to one aspect of ribs 70, 72, the ribs 70, 72 extend radially outward a desired distance from the inter-rib region 74 of cylindrical portion 58 (i.e., a rib thickness) to act as bearing points against the inner surface of the syringe barrel 12. In some embodiments, an outer diameter of the ribs, $OD_{ribs}$, is controlled based on an inner diameter of barrel 12, $ID_{barrel}$, (see FIG. 1) and to provide a desired amount of interference between the stopper 38 and the barrel 12. As one example, the rib thickness and the outer diameter of the ribs, $OD_{ribs}$, is such that an interference between the stopper 38 and the syringe barrel 12 is in the range of 1.5 to 20%, as determined by: Interference = $(OD_{nbs}/ID_{barrel}) - 1) \times 100$. According to another aspect of ribs 70, 72, a width of each of the first rib 70 and the second rib 72 that makes contact with the syringe barrel 12 (i.e., a radially outward-directed contact surface 76 of ribs 70, 72) is between 0.2 and 10 mm, and preferably between 0.2 and 1.5 mm.

According to aspects of the disclosure, the first rib 70 and the second rib 72 are formed on stopper 38 so as to be spaced apart by at least a minimum axial distance (i.e., a minimum pitch) as compared to the overall axial length of the stopper 38. That is, a length of the inter-rib region 74, $L_{inter-rib}$, as measured between a proximal edge of the first (distal) rib 70 and a distal edge of the second (proximal) rib 72, is configured to form a minimum overall percentage of the main body axial length, $L_{body}$, of stopper 38. According to some embodiments, the first and second ribs 70, 72 are axially spaced apart such that the inter-rib axial length, $L_{inter-rib}$, of the inter-rib region 74 is 30% or greater of the overall body axial length, $L_{body}$. In certain configurations, the inter-rib axial length is preferably 70% or greater (i.e., 70%-100%) of the body axial length, $L_{body}$. Provided here below are several non-limiting examples of inter-rib axial lengths for stoppers of various sizes/configuration.

In a first example, where the syringe 10 is provided as a 10 mL syringe, the stopper 38 may be formed to have a main body axial length, $L_{body}$, of approximately 9 mm (e.g., 9 mm +/- 0.5 mm). According to aspects of the disclosure, with stopper 38 having a main body axial length, $L_{body}$, of 9 mm, the first rib 70 and the second rib 72 should be axially spaced apart such that the inter-rib axial length, $L_{inter-rib}$, of the inter-rib region is 3 mm or greater (which is ≥ 30% of the main body axial length, $L_{body}$), and preferably between 6 and 8 mm.

In a second example, where the syringe 10 is provided as a 5 mL syringe, the stopper 38 may be formed to have a main body axial length, $L_{body}$, of approximately 8.5 mm (e.g., 8.5 mm +/-0.5 mm). According to aspects of the disclosure, with stopper 38 having a main body axial length, $L_{body}$, of 8.5 mm, the first rib 70 and the second rib 72 should be axially spaced apart such that the inter-rib axial length, $L_{inter-rib}$, of the inter-rib region is 2.9 mm or greater (which is ≥ 30% of the main body axial length, $L_{body}$), and preferably between 5.5 and 7.5 mm.

In a third example, where the syringe 10 is provided as a 1-3 mL syringe, the stopper 38 may be formed to have a main body axial length, $L_{body}$, of approximately 7.7 mm (e.g., 7.7 mm +/-0.5 mm). According to aspects of the disclosure, with stopper 38 having a main body axial length, $L_{body}$, of 7.7 mm, the first rib 70 and the second rib 72 should be axially spaced apart such that the inter-rib axial length, $L_{inter-rib}$, of the inter-rib region is 2.6 mm or greater (which is ≥ 30% of the main body axial length, $L_{body}$), and preferably between 5 and 7.7 mm.

With the first rib 70 and the second rib 72 spaced axially apart along the main body 52 by at least a minimum axial distance/pitch as compared to the main body axial length, $L_{body}$, of stopper 38, the ribs function as a stabilizing feature configured to maintain an axial guiding and aligning of the stopper 38 within the syringe barrel 12. That is, it is recognized that there is a risk that a stopper may tilt or become misaligned as it is initially stoppered within syringe barrel 12 (via a vent tube stoppering process where an insertion rod pushes the stopper out of a vent tube and into the syringe barrel 12) or is advanced distally through the syringe barrel 12 during use of syringe 10 (responsive to a distally directed actuation/pushing of the plunger rod 36), and the spacing apart of ribs 70, 72 on stopper 38 help to prevent such tilting/misalignment. When the pitch between the ribs 70, 72 is sized so as to be at least 30% or more of the overall length of the stopper (i.e., inter-rib axial length, $L_{inter-rib}$, is ≥ 30% of the main body axial length, $L_{body}$), such tilting/misalignment of the stopper 38 may be prevented due to the stopper 38 having a greater axial contact area with the syringe barrel 12, which enables the stopper 38 to maintain better isostatism with the syringe barrel 12. With the axial guiding and aligning of the stopper 38 within the syringe barrel 12 being maintained, the ribs 70, 72 of stopper 38 are able to apply a consistent contact pressure against syringe barrel 12, so as to guarantee proper sealing therewith and maintain leak resistance and container closure integrity in the syringe 10.

In addition to the first rib 70 and the second rib 72 functioning as a stabilizing feature configured to maintain an axial guiding and aligning of the stopper 38 within the syringe barrel 12, the inclusion of only a first rib 70 and second rib 72 on the stopper 38 also functions to limit the amount of friction created between the stopper 38 and the syringe barrel 12. That is, while the stopper 38 provides guiding and alignment features comparable to a three-ribbed stopper (due to an increased pitch between ribs 70, 72), the inclusion of only two ribs 70, 72 on stopper 38 serves to minimize the amount of friction present between the stopper 38 and syringe barrel 12 during advancement of the stopper 38 therethrough (as part of plunger assembly 14) - with the amount of friction being less than found on a three-ribbed stopper.

Beneficially, embodiments of the invention thus are directed to a stopper having spaced apart first and second ribs formed thereon that extend from an outer surface of a main body portion of the stopper and extend around an outer circumference of the main body portion. An inter-rib region of the outer surface is present between the first and second ribs, such that the ribs have a pitch therebetween, with the inter-rib region having an axial length that is 30% or more of the overall length of the stopper - with this pitch ensuring that proper alignment of the stopper within the syringe barrel is maintained during placement/advancement thereof. The inclusion of only first and second ribs on the stopper also controls/lowers the amount of friction present between the stopper and syringe barrel during advancement of the stopper therethrough.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment may be combined with one or more features of any other embodiment.

**Claims**

1. A stopper adapted for attachment with a plunger rod for use within a syringe barrel, the stopper comprising:

   a main body portion comprising a proximal end and a distal end and having a main body axial length extending between the proximal and distal ends, the proximal end configured to mate with a distal end of the plunger rod to secure the stopper to the plunger rod;
   a first rib extending radially from an outer surface of the main body portion and around an outer circumference of the main body portion;
   a second rib axially spaced from the first rib, the second rib extending radially from the outer surface of the main body portion and around the outer circumference of the main body portion; and

wherein the main body portion includes an inter-rib region extending axially between the first rib and the second rib, **characterized in that** an inter-rib axial length of the inter-rib region is 30 percent or greater of the main body axial length.

2. The stopper of claim 1, wherein the inter-rib axial length is preferably 70 percent or greater of the main body axial length.

3. The stopper of claim 1 or claim 2, wherein the first rib is a distal rib and the second rib is a proximal rib, and wherein the inter-rib axial length comprises a pitch between a proximal edge of the distal rib and a distal edge of the proximal rib.

4. The stopper of claim 3, wherein the first rib and the second rib, and the pitch therebetween, forms a stabilizing feature configured to maintain an axial guiding and aligning of the stopper within the syringe barrel.

5. The stopper of any of claims 1-4, wherein each of the first rib and the second rib comprises a radially outward-directed contact surface, and wherein a width of the contact surface is between 0.2 mm and 10 mm and preferably between 0.2 mm and 1.5 mm.

6. The stopper of any of claims 1-5, wherein the first and second ribs are integrally formed with the main body portion, and wherein each of the main body portion and the first and second ribs are formed of a base material, the base material comprising rubber.

7. The stopper of any of claims 1-6, further comprising a laminate or coating applied to at least a radially outward-directed contact surface of the first rib and the second rib, the laminate or coating having a lower coefficient of friction than the base material.

8. The stopper of claim 7, wherein the laminate or coating comprises one or more of ultra-high-molecular-weight polyethylene (UHMWPE), liquid silicone rubber (LSR), polydicyclopentadiene (PDCPD), polymethyl urea (PMU), polyvinylidene difluoride (PVDF), polyvinyl, a polysulfone film, or a fluoropolymer.

9. A syringe, comprising:

a syringe barrel having a barrel proximal end and a barrel distal end and defining a chamber configured for containing a fluid therein; and
a plunger assembly axially movable within the chamber of the syringe barrel between a retracted position and an advanced position, the plunger assembly including:

a plunger rod having a plunger proximal end and a plunger distal end; and
the stopper of claim 1 coupled to the plunger distal end.

10. The syringe of claim 9, wherein the inter-rib axial length is preferably 70 percent or greater of the main body axial length, so as to prevent tilting of the stopper when placed into or advanced through the syringe barrel.

11. The syringe of claim 9 or claim 10, wherein the syringe comprises a 10 mL syringe, with the main body axial length of the stopper being approximately 9 mm and the inter-rib axial length of the inter-rib region being between 6 and 9 mm.

12. The syringe of claim 9 or claim 10, wherein the syringe comprises a 5 mL syringe, with the main body axial length of the stopper being approximately 8.5 mm and the inter-rib axial length of the inter-rib region being between 5 and 8.5 mm.

13. The syringe of claim 10 or claim 11, wherein the syringe comprises a 1-3 mL syringe, with the main body axial length of the stopper being approximately 7.7 mm and the inter-rib axial length of the inter-rib region being between 5 and 7.7 mm.

14. The syringe of any of claims 10-13, wherein the stopper comprises a base material and a laminate or coating applied to at least a portion of an outer surface of the base material, wherein the base material comprises rubber, and wherein the laminate or coating comprises one or more of ultra-high-molecular-weight polyethylene (UHMWPE), liquid silicone rubber (LSR), polydicyclopentadiene (PDCPD), polymethyl urea (PMU), polyvinylidene difluoride (PVDF), polyvinyl, a polysulfone film, or a fluoropolymer.

15. The syringe of any of claims 10-14, wherein an interference between the first and second ribs and the syringe barrel is in the range of 1.5 to 20%, as determined by:

$$\text{Interference} = ((OD_{ribs}/ID_{barrel}) - 1) \times 100,$$

wherein $OD_{ribs}$ is an outer diameter of the first and second ribs and $ID_{barrel}$ is an inner diameter of the syringe barrel.

FIG. 1

FIG. 2

FIG. 3

FIG. 4

FIG. 5
(PRIOR ART)

FIG. 6
(PRIOR ART)

Europäisches
Patentamt
European
Patent Office
Office européen
des brevets

## EUROPEAN SEARCH REPORT

Application Number

EP 24 18 5252

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/046248 A1 (HETTING MIKAEL [DK]) 18 February 2021 (2021-02-18) * paragraph [0015]; figures 2-4 * * paragraph [0018] * * paragraph [0021] * * paragraph [0026] * * paragraph [0028] * ----- | 1-6, 9-13,15 | INV. A61M5/315 |
| X | EP 2 226 088 A1 (TERUMO CORP [JP]) 8 September 2010 (2010-09-08) * paragraph [0003]; figures 2,5 * * paragraph [0009] - paragraph [0010] * * paragraph [0023] * ----- | 1,3-9, 11-14 | |
| X | US 2014/062036 A1 (MAEDA KATSUSHI [JP] ET AL) 6 March 2014 (2014-03-06) * paragraph [0012] * * paragraph [0015] - paragraph [0016] * * paragraph [0025]; figure 1 * * paragraph [0029] * * paragraph [0042] * * paragraph [0044] * ----- | 1-15 | |
| | | | TECHNICAL FIELDS SEARCHED (IPC) |
| A | EP 0 338 671 A1 (DAIKYO GOMU SEIKO KK [JP]) 25 October 1989 (1989-10-25) * paragraph [0009]; figure 2 * * paragraph [0011] * * paragraph [0014] * ----- | 1-15 | A61M |

The present search report has been drawn up for all claims

| Place of search | Date of completion of the search | Examiner |
|---|---|---|
| Munich | 14 October 2024 | Grialou, Cédric |

EPO FORM 1503 03.82 (P04C01)

# EP 4 670 761 A1

## ANNEX TO THE EUROPEAN SEARCH REPORT
## ON EUROPEAN PATENT APPLICATION NO.

EP 24 18 5252

This annex lists the patent family members relating to the patent documents cited in the above-mentioned European search report.
The members are as contained in the European Patent Office EDP file on
The European Patent Office is in no way liable for these particulars which are merely given for the purpose of information.

14-10-2024

| Patent document cited in search report | | Publication date | Patent family member(s) | | Publication date |
|---|---|---|---|---|---|
| US 2021046248 | A1 | 18-02-2021 | AU | 2019241406 A1 | 22-10-2020 |
| | | | BR | 112020019255 A2 | 12-01-2021 |
| | | | CA | 3095075 A1 | 03-10-2019 |
| | | | CN | 112041010 A | 04-12-2020 |
| | | | EP | 3773821 A1 | 17-02-2021 |
| | | | IL | 277603 A | 30-11-2020 |
| | | | JP | 7321535 B2 | 07-08-2023 |
| | | | JP | 2021519136 A | 10-08-2021 |
| | | | KR | 20210013547 A | 04-02-2021 |
| | | | RU | 2020133560 A | 27-04-2022 |
| | | | SG | 11202009433V A | 29-10-2020 |
| | | | US | 2021046248 A1 | 18-02-2021 |
| | | | WO | 2019185101 A1 | 03-10-2019 |
| | | | ZA | 202006070 B | 26-01-2022 |
| EP 2226088 | A1 | 08-09-2010 | CN | 101909675 A | 08-12-2010 |
| | | | EP | 2226088 A1 | 08-09-2010 |
| | | | ES | 2886499 T3 | 20-12-2021 |
| | | | JP | 5394256 B2 | 22-01-2014 |
| | | | JP | 5791683 B2 | 07-10-2015 |
| | | | JP | 2014036876 A | 27-02-2014 |
| | | | JP | WO2009084646 A1 | 19-05-2011 |
| | | | US | 2010324501 A1 | 23-12-2010 |
| | | | US | 2015133873 A1 | 14-05-2015 |
| | | | WO | 2009084646 A1 | 09-07-2009 |
| US 2014062036 | A1 | 06-03-2014 | CN | 103656803 A | 26-03-2014 |
| | | | EP | 2703025 A1 | 05-03-2014 |
| | | | JP | 5960554 B2 | 02-08-2016 |
| | | | JP | 2014047828 A | 17-03-2014 |
| | | | US | 2014062036 A1 | 06-03-2014 |
| EP 0338671 | A1 | 25-10-1989 | DE | 68915328 T2 | 25-08-1994 |
| | | | EP | 0338671 A1 | 25-10-1989 |
| | | | JP | H0534669 Y2 | 02-09-1993 |
| | | | JP | H01138454 U | 21-09-1989 |
| | | | US | 5009646 A | 23-04-1991 |

EPO FORM P0459

For more details about this annex : see Official Journal of the European Patent Office, No. 12/82

14